# EUROPEAN PATENT SPECIFICATION

(11) **EP 1 945 757 B1**
(45) Date of publication and mention of the grant of the patent: **06.12.2017**
(21) Application number: 06817074.5
(22) Date of filing: 17.10.2006
(51) Int. Cl.: A61L 27/60, A61L 27/38

(54) **METHOD OF DELIVERING HAIR FOLLICLE PROGENITOR CELLS TO THE SKIN**
METHODE ZUR VERTEILUNG VON HAARFOLLIKEL-VORLÄUFERZELLEN AUF DER HAUT
PROCÉDÉ CONSISTANT À INSÉRER DES CELLULES DANS LA PEAU

(30) Priority: 17.10.2005 US 727588 P
(43) Date of publication of application: 23.07.2008
(73) Proprietor: Aderans Research Institute, Inc., Beverly Hills, CA 90212 (US)
(72) Inventor: ZHENG, Ying, Wayne, PA 19087 (US); DU, Xiaobing, Philadelphia, PA 19103 (US); WASHENIK, Kenneth, Justin, Los Angeles, CA 90046 (US); STENN, Kurt, Stricker, Princeton, NJ 08540 (US)
(74) Representative: Hoffmann Eitle
(86) International application number: PCT/US2006/040608
(87) International publication number: WO 2007/047707

(56) References cited:
- EP-A1- 0 236 014
- EP-A1- 1 437 042
- WO-A-99/01034
- WO-A-03/068248
- WO-A-2005/018731
- WO-A2-02/060396
- US-A1- 2002 106 353
- JAHODA C A B ET AL: "DERMAL-EPIDERMAL INTERACTIONS. ADULT FOLLICLE-DERIVED CELL POPULATIONS AND HAIR GROWTH" DERMATOLOGIC CLINICS, W.B. SAUNDERS CO., LONDON, GB, vol. 14, no. 4, October 1996 (1996-10), pages 573-583, XP002913549 ISSN: 0733-8635

## Description

### BACKGROUND OF THE INVENTION

Developing appropriate cell delivery techniques is important for many applications. Not only must the cells be delivered to the appropriate part of the subject, but the morphology of the cells must be maintained. The morphology of cells can be important in many contexts. Cell morphology indicates the status of the cells, both in terms of the health of the cells and in terms of the differentiation state of the cell. Changes in cell shape, or morphogenesis, are central to cell function, development and disease. Physiological processes in cells are often accompanied by changes in cellular morphology. Examples include changes in the intracellular location, arrangement and structure of cellular constituents, such as organelles, macromolecular clusters or the cytoskeleton, changes in the morphology of the entire cell, such as its shape and area, changes in the spacing and proximity between cells, and properties of multi-cellular colonies such as its shape, size and cell locations.

### SUMMARY OF THE INVENTION

The present invention relates to the use of skin forming cells for the manufacture of a medicament and skin forming cells for use in a method of treating, repairing or improving a condition selected from the group consisting of skin ulcers, diabetic foot ulcers, bed sores, burn wounds, microbial infections, and scars, wherein the method comprises making a wound in the skin with a sharp instrument so that the wound is shallow enough that the subject does not bleed; and placing the skin forming cells on the wound, wherein new skin is formed.

The invention is disclosed in the claims.

### BRIEF DESCRIPTION OF THE FIGURES

FIG. 1 shows a grid of superficial cuts on the surface of the skin of a nude mouse and hair out-growth (inset) observed at two weeks post-administration with black newborn mouse skin cells.
FIG. 2 shows a scalpel blade being used to make a superficial stab wound on the surface of the skin of a nude mouse and hair out-growth (inset) observed at two weeks post-administration of black newborn mouse skin cells into the wound.

### DETAILED DESCRIPTION OF THE INVENTION

It has been discovered that cells can be delivered into the superficial skin without disturbing the inherent characteristics of the cells by making a wound in an area where the cells are desired and placing the cells on the wound. It is expected that the cells would orientate themselves appropriately to form the desired structure upon delivery with the method of the present invention. Suitably, the cells can be placed on the surface of the skin on top of the wound or in the wound itself.

The cell delivery method of the present disclosure can be used to form egressing hair shafts. The cell delivery method may also be used to form new skin to treat, repair or improve conditions including, but not limited to, skin ulcers, diabetic foot ulcers, bed sores, burn wounds, microbial infections, and scars such as those resulting from surgery, acne, or illnesses such as chicken pox. Delivery of appropriate cells by the methods of the present disclosure could also be used to form sweat glands, nail, eyebrow, eyelash and other hairs. The method of the present disclosure could also be used to form the dermis or epidermis using genetically altered autologous or allogenic cells. Suitably, the cell delivery method may be used to form both the dermal and epidermal layers of the skin. Alternatively, either the dermal or the epidermal layers may be formed by the disclosed method.

The wound may be formed by any technique that disrupts the outer surface of the area in which the cells are desired such as stabbing, cutting or scratching the area with a sharp instrument, including, but not limited to, a scalpel blade or a needle. The wound may also be formed by abrading the area with, e.g. needles such as microneedles or grooved needles.

For certain embodiments, the sharp instrument may be modified to form a wound with a measured depth. For example, the wound may be at least about 10 µm in depth, at least about 200 µm in depth, no more than about 500 µm in depth, or no more than about 250 µm in depth. The wound may be from about 10 µm to about 500 µm in depth. The wound may be unlimited in maximal length. In certain embodiments of the invention, the depth of the wound may be adjusted to deliver the cells into the subepidermis, the papillary dermis, the upper reticular dermis, or the same levels of the nail bed of the acral skin (palmer-plantar skin). The wound is shallow enough that the subject does not bleed. The wound suitably heals without forming a scar.

After the cells are placed on the wound, the wound may be covered with a bandage or dressing; for example, a non-adhering dressing, or a transparent plastic dressing such as Tegaderm® (3M, St. Paul, MN) or a gel-based burn dressing. Petroleum jelly or the like may also be applied to the wound. The dressing may be left in place for about 3 to about 7 days. Suitably, the dressing may be substantially water-impermeable.

The cells may comprise dermal cells, epidermal cells, epidermal stem cells, basal cells, keratinocytes, fibroblasts or combinations thereof. The cells may be derived from follicular, eccrine or nail sources. Suitably, the cells are skin forming cells or hair follicle progenitor cells. For certain embodiments of the present invention, the ratio of epidermal to dermal cells is suitably about 10:1 to about 1:10. Suitably, the ratio may be about 1:1 to about 5:1.

The cells may suitably be provided in a suspension in a physiologically acceptable carrier, e.g., sterile saline solution. Additional components may also be added to the cell suspension. Suitable additional components include growth factors, nutrient molecules or stabilizing molecules.

The following examples are provided to assist in further understanding of the invention.

### EXAMPLES

### Example 1. Hair outgrowth from follicle-inductive cells administered to superficial cuts on the surface of mouse skin (not according to the invention).

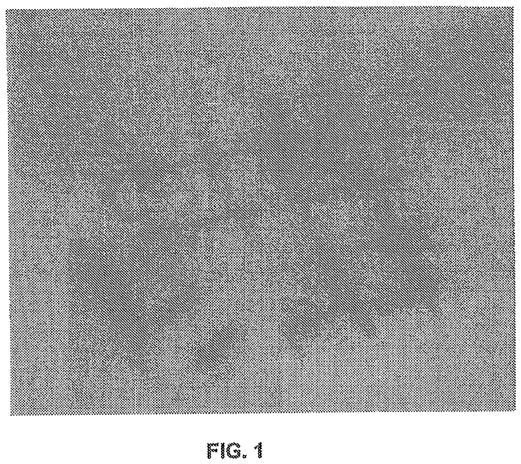

An athymic nude (*nu*/*nu*) mouse (Charles River, Inc.) was anesthetized and a grid of superficial cuts (Figure 1) was made on the dorsal skin with the use of a number 11 scalpel blade. The cuts were shallow enough not to draw blood. A mixture of freshly isolated newborn black mouse skin cells comprising 100,000 epidermal cells and 200,000 dermal cells was prepared as follows. Truncal skin was removed from newborn mice and rinsed in Ca²⁺ and Mg²⁺ free PBS. The skin was laid flat in PBS containing Dispase (2.5 mg per ml, Invitrogen, Carslbad, CA) at 4°C overnight or 37°C for 2 hours. Inductive dermal and epidermal cells were isolated as described in Weinberg et al., J. Invest. Dermatol., 100:229-236 (1993). A suspension of the cells in 2 microliters of sterile buffered saline solution was delivered to the grid-of-cuts by pipette. The skin was gently pulled apart for a few seconds to allow the fluid to wick throughout the grid. A non-adherent, hydrophobic (petrolatum coated gauze) dressing was applied to the wound and the mouse was further bandaged with an elastic wrap to prevent removal of the dressing upon recovery from anesthesia. The dressing was removed after 10 days. After 2 weeks the growth of hair was observed (Figure 1, inset) primarily within the grid pattern. The mouse was then euthanized and the skin gently removed. Observation of the underside of the skin revealed almost no ingrown hairs and the presence of follicle bulbs corresponding to the visible hair seen on the skin surface.

### Example 2. Hair outgrowth from follicle-inductive cells administered to superficial stab wound on the surface of mouse skin (not according to the invention).

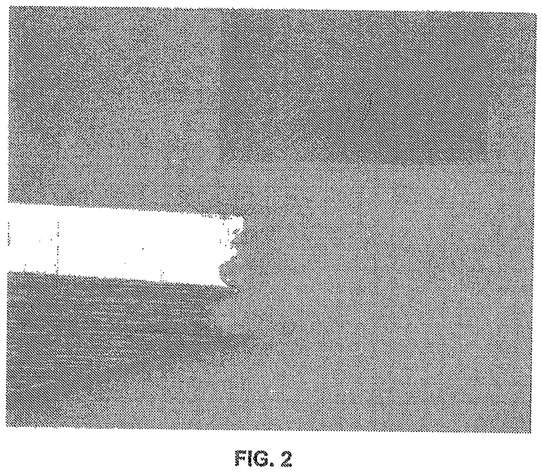

An athymic nude (*nu*/*nu*) mouse (Charles River, Inc.) was anesthetized and a stab wound was made in the skin by piercing with the tip of a number 11 scalpel blade held a low angle to the surface of the skin (Figure 2). A mixture of freshly isolated newborn black mouse skin cells comprising 100,000 epidermal cells and 200,000 dermal cells was prepared as described above. A suspension of the cells in 2 microliters of sterile buffered saline solution was instilled into the cut by pipette. A non-adherent, hydrophobic (petrolatum coated gauze) dressing was applied to the wound and the mouse was further bandaged with an elastic wrap to prevent removal of the dressing upon recovery from anesthesia. The dressing was removed after 10 days. After 2 weeks the growth of hair was observed (Figure 2, inset) precisely at the site of the stab incision. The mouse was then euthanized and the skin gently removed. Observation of the underside of the skin revealed almost no ingrown hairs and the presence of follicle bulbs corresponding to the visible hair seen on the skin surface.

### Example 3. Use of different wound dressing materials (not according to the invention).

Examples 1 and 2 were repeated using different types of wound dressing materials as shown in Table 1.

**TABLE 1**

| **Superficial Delivery of Mouse Cells into Nude Mice** | | | | | |
|---|---|---|---|---|---|
| **Delivery Method** | **Type of Dressing** | **# of Newborn Black Mouse Skin Cells Delivered** | **Vol. (µL)** | **Number of Replicates** | **Outgrowth Rate** |
| Example 1 | None | 1 x 10⁵ Epidermal mixed with 2 x 10⁵ Dermal | 2 | 8 | 0 |
| Example 1 | Non-adhering | 1 x 10⁵ Epidermal mixed with 2 x 10⁵ Dermal | 2 | 6 | 6 |
| Example 1 | Burn pad | 1 x 10⁵ Epidermal mixed with 2 x 10⁵ Dermal | 2 | 4 | 3 |
| Example 1 | Tegaderm™ | 1 x 10⁵ Epidermal mixed with 2 x 10⁵ Dermal | 2 | 5 | 2 |
| Example 2 | None | 1 x 10⁵ Epidermal mixed with 2 x 10⁵ Dermal | 2 | 12 | 5 |
| Example 2 | Non-adhering | 1 x 10⁵ Epidermal mixed with 2 x 10⁵ Dermal | 2 | 16 | 9 |
| Example 2 | Burn pad | 1 x 10⁵ Epidermal mixed with 2 x 10⁵ Dermal | 2 | 5 | 5 |
| Example 2 | Tegaderm™ | 1 x 10⁵ Epidermal mixed with 2 x 10⁵ Dermal | 2 | 5 | 2 |

### Example 4. New hair growth in bald scalp by application of autologous cells into superficial incisions (not according to the invention).

Superficial wounds are created on a human subject's head with a surgical blade or scalpel, as grids, multiple crosses, parallel lines or similar such pattern to achieve the desired cosmetic effect for hair growth. Autologous human trichogenic dermal and epidermal cells (optionally mixed with hair follicle melanocytes if needed to restore hair pigmentation) in suspension are then spread on top of the superficial cuts with pipette tips or syringes and the wound is covered for 2-3 days to prevent the cells from drying out prior to being incorporated into the skin. Egressing hair follicles are then formed.

### Example 5. New hair growth in bald scalp by application of dermal cells into superficial incisions (not according to the invention).

Wounds are created on a human subject's head with a surgical blade or scalpel, as grids, multiple crosses, parallel lines or similar such pattern to achieve the desired cosmetic effect for hair growth. Human trichogenic dermal cells alone (optionally mixed with hair follicle melanocytes if needed to restore hair pigmentation) in suspension are then spread on top of the superficial cuts with pipette tips or syringes and the wound is covered for 2-3 days to prevent the cells from drying out prior to being incorporated into the skin. Egressing hair follicles are then formed.

### Example 6. Formation of new skin to treat a subject with a diabetic foot ulcer.

A subject with a diabetic foot ulcer is anesthetized under local or general anesthesia and a series of superficial cuts is made in the ulcer. The cuts are shallow enough not to draw blood. A suspension of basal cells in 2 microliters of sterile buffered saline solution is delivered to the cuts by pipette. The skin is gently pulled apart for a few seconds to allow the fluid to wick throughout the grid. A non-adherent, hydrophobic (petrolatum coated gauze) dressing is applied to the wound. The dressing is removed after 10 days. New skin forms and the ulcer is healed.

### Example 7. Formation of new skin to treat a subject with bed sores.

A subject with bed sores is anesthetized under local or general anesthesia and a series of superficial cuts is made in the bed sore. The cuts are shallow enough not to draw blood. A suspension of basal cells in 2 microliters of sterile buffered saline solution is delivered to the cuts by pipette. The skin is gently pulled apart for a few seconds to allow the fluid to wick throughout the grid. A non-adherent, hydrophobic (petrolatum coated gauze) dressing is applied to the wound. The dressing is removed after 10 days. New skin forms and the bed sore is healed.

## Claims

1. Use of skin forming cells for the manufacture of a medicament for the treatment, repair or improvement of a condition selected from the group consisting of skin ulcers, diabetic foot ulcers, bed sores, burn wounds, microbial infections, and scars,
wherein the treatment, repair or improvement of the condition comprises making a wound in the skin with a sharp instrument so that the wound is shallow enough that the subject does not bleed; and placing the skin forming cells on the wound, wherein new skin is formed.

2. The use of claim 1, wherein the cells comprise basal cells.

3. The use of claim 1, wherein the cells are placed in subepidermal skin, or in papillary dermal skin, or in upper reticular dermal skin.

4. The use of claim 1, wherein the wound is at least about 10 µm in depth, or at least about 200 µm in depth, or no more than about 500 µm in depth, or no more than about 250 µm in depth.

5. Skin forming cells for use in a method of treating, repairing or improving a condition selected from the group consisting of skin ulcers, diabetic foot ulcers, bed sores, burn wounds, microbial infections, and scars,
wherein the method comprises making a wound in the skin with a sharp instrument so that the wound is shallow enough that the subject does not bleed; and placing the skin forming cells on the wound, wherein new skin is formed.

6. The skin forming cells for use according to claim 5, wherein the cells comprise basal cells.

7. The skin forming cells for use according to claim 5, wherein the cells are placed in subepidermal skin, or in papillary dermal skin, or in upper reticular dermal skin.

8. The skin forming cells for use according to claim 5, wherein the wound is at least about 10 µm in depth, or at least about 200 µm in depth, or no more than about 500 µm in depth, or no more than about 250 µm in depth.

## Patentansprüche

1. Verwendung von hautbildenden Zellen für die Herstellung eines Medikaments zur Behandlung, Behebung oder Verbesserung eines Zustands, ausgewählt aus der Gruppe, bestehend aus Hautgeschwüren, diabetischen Fußgeschwüren, Druckgeschwüren, Brandwunden, mikrobiellen Infektionen und Narben,
wobei die Behandlung, Behebung oder Verbesserung des Zustands umfasst: das Schaffen einer Wunde mit einem scharfen Instrument, sodass die Wunde flach genug ist, dass das Subjekt nicht blutet; und das Anbringen der hautbildenden Zellen auf der Wunde, wobei neue Haut gebildet wird.

2. Verwendung gemäß Anspruch 1, wobei die Zellen Basalzellen umfassen.

3. Verwendung gemäß Anspruch 1, wobei die Zellen in subepidermaler Haut oder in papillärer dermaler Haut oder in oberer retikularer dermaler Haut angebracht werden.

4. Verwendung gemäß Anspruch 1, wobei die Wunde wenigstens etwa 10 µm tief oder wenigstens etwa 200 µm tief oder nicht mehr als etwa 500 µm tief oder nicht mehr als etwa 250 µm tief ist.

5. Hautbildende Zellen zur Verwendung beim Behandeln, Beheben oder Verbessern eines Zustands, ausgewählt aus der Gruppe, bestehend aus Hautgeschwüren, diabetischen Fußgeschwüren, Druckgeschwüren, Brandwunden, mikrobiellen Infektionen und Narben,
wobei die Behandlung, Behebung oder Verbesserung des Zustands umfasst: das Schaffen einer Wunde mit einem scharfen Instrument, sodass die Wunde flach genug ist, dass das Subjekt nicht blutet; und das Anbringen der hautbildenden Zellen auf der Wunde, wobei neue Haut gebildet wird.

6. Hautbildende Zellen zur Verwendung gemäß Anspruch 5,
wobei die Zellen Basalzellen umfassen.

7. Hautbildende Zellen zur Verwendung gemäß Anspruch 5,
wobei die Zellen in subepidermaler Haut oder in papillärer dermaler Haut oder in oberer retikularer dermaler Haut angebracht werden.

8. Hautbildende Zellen zur Verwendung gemäß Anspruch 5,
wobei die Wunde wenigstens etwa 10 µm tief oder wenigstens etwa 200 µm tief oder nicht mehr als etwa 500 µm tief oder nicht mehr als etwa 250 µm tief ist.

## Revendications

1. Utilisation de cellules de formation de peau pour la fabrication d'un médicament pour le traitement, la réparation ou l'amélioration d'une pathologie sélectionnée dans le groupe constitué des ulcères de la peau, des ulcères du pied diabétiques, des escarres, des plaies de brûlure, des infections microbiennes, et des cicatrices, dans laquelle le traitement, la réparation ou l'amélioration de la pathologie comprend la réalisation d'une plaie dans la peau avec un instrument tranchant de sorte que la plaie soit suffisamment peu profonde pour que le sujet ne saigne pas ; et le placement les cellules de formation de peau sur la plaie, dans laquelle de la nouvelle peau est formée.

2. Utilisation selon la revendication 1, dans laquelle les cellules comprennent des cellules basales.

3. Utilisation selon la revendication 1, dans laquelle les cellules sont placées dans la peau sous-épidermique, ou dans la peau dermique papillaire, ou dans la peau dermique réticulaire supérieure.

4. Utilisation selon la revendication 1, dans laquelle la plaie est d'au moins environ 10 I1m en profondeur, ou d'au moins environ 200 µm en profondeur, ou de pas plus d'environ 500 µm en profondeur, ou de pas plus d'environ 250 µm en profondeur.

5. Cellules de formation de peau pour une utilisation dans une méthode de traitement, de réparation ou d'amélioration d'une pathologie sélectionnée dans le groupe constitué des ulcères de la peau, des ulcères du pied diabétiques, des escarres, des plaies de brûlure, des infections microbiennes, et des cicatrices, dans lesquelles la méthode comprend la réalisation d'une plaie dans la peau avec un instrument tranchant de sorte que la plaie soit suffisamment peu profonde pour que le sujet ne saigne pas; et le placement des cellules de formation de peau sur la plaie, dans laquelle de la nouvelle peau est formée.

6. Cellules de formation de peau pour une utilisation selon la revendication 5, dans lesquelles les cellules comprennent des cellules basales.

7. Cellules de formation de peau pour une utilisation selon la revendication 5, dans lesquelles les cellules sont placées dans la peau sous-épidermique, ou dans la peau dermique papillaire, ou dans la peau dermique réticulaire supérieure.

8. Cellules de formation de peau pour une utilisation selon la revendication 5, dans lesquelles la plaie est d'au moins environ 10 µm en profondeur, ou d'au moins environ 200 µm en profondeur, ou de pas plus d'environ 500 µm en profondeur, ou de pas plus d'environ 250 µm en profondeur.
